Europäisches Patentamt

European Patent Office

Office européen des brevets

(19)

(11) Veröffentlichungsnummer: **0 615 440 B1**

(12) **EUROPÄISCHE PATENTSCHRIFT**

(45) Veröffentlichungstag der Patentschrift: **13.09.95**

(51) Int. Cl.6: **A61K 9/10**, A61K 9/51, A61K 31/19

(21) Anmeldenummer: **92924549.6**

(22) Anmeldetag: **04.12.92**

(86) Internationale Anmeldenummer: **PCT/DE92/01012**

(87) Internationale Veröffentlichungsnummer: **WO 93/10761 (10.06.93 93/14)**

(54) **EIN 2-ARYLPROPIONSÄUREDERIVAT IN NANOSOLFORM ENTHALTENDES ARZNEIMITTEL UND SEINE HERSTELLUNG.**

(30) Priorität: **05.12.91 DE 4140185**

(43) Veröffentlichungstag der Anmeldung:
**21.09.94 Patentblatt 94/38**

(45) Bekanntmachung des Hinweises auf die Patenterteilung:
**13.09.95 Patentblatt 95/37**

(84) Benannte Vertragsstaaten:
**AT BE CH DE DK ES FR GB GR IT LI NL PT SE**

(56) Entgegenhaltungen:
EP-A- 0 275 796      EP-A- 0 282 020
WO-A-85/04099      WO-A-89/04658
WO-A-91/06292      FR-A- 2 608 427

(73) Patentinhaber: **ALFATEC-PHARMA GmbH**
**Im Neuenheimer Feld 519**
**D-69120 Heidelberg (DE)**

Patentinhaber: **PAZ ARZNEIMITTEL- ENTWICK-
LUNGSGESELLSCHAFT MBH
In der Schildwacht 13**

D-65933 Frankfurt (DE)

(72) Erfinder: **WUNDERLICH, Jens-Christian
Bothestrasse 52
D-6900 Heidelberg (DE)**
Erfinder: **SCHICK, Ursula
Staatsbahnhofstrasse 6
D-6908 Wiesloch (DE)**
Erfinder: **FREIDENREICH, Jürgen
Huberweg 26
D-6905 Schriesheim (DE)**
Erfinder: **WERRY, Jürgen
Weimarer Strasse 20
D-6700 Ludwigshafen (DE)**
Erfinder: **LUKAS, Helmut
Taunusstrasse 30
D-6078 Neu-Isenburg (DE)**
Erfinder: **SCHUSTER, Otto
Kelkheimerstrasse 69
D-6232 Bad Soden (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

(74) Vertreter: **Fürniss, Peter, Dr.**
**Kuhnen, Wacker & Partner**
**Alois-Steinecker-Strasse 22**
**D-85354 Freising (DE)**

**Beschreibung**

Die Erfindung betrifft ein Akut-Arzneimittel zur Behandlung von schmerzhaften und/oder entzündlichen, sowie fieberhaften Erkrankungen, das als Wirkstoff ein 2-Arylpropionsäurederivat in Form eines pharmazeutisch applizierbaren Nanosols und als Träger im wesentlichen Gelatine, ein Kollagenhydrolysat oder ein Gelatinederivat neben üblichen pharmazeutischen Hilfsstoffen enthält.

Die Erfindung betrifft weiterhin die Verwendung eines pharmazeutisch applizierbaren Nanosols von einem 2-Arylpropionsäurederivat zur Herstellung von Arzneimitteln mit akuter analgetischer und/oder antirheumatischer, sowie antipyretischer Wirkung.

Die Erfindung betrifft schließlich ein Verfahren zur Herstellung eines kolloid-dispersen Systems eines 2-Arylpropionsäurederivates.

Nichtsteroidale Antirheumatika (NSAR), wie beispielsweise die Wirkstoffe aus der Substanzklasse der Salicylsäurederivate (Acetylsalicylsäure), Indolylessigsäurederivate (Indometacin), Phenylessigsäurederivate (Diclofenac), Oxicame (Piroxicam) werden zur symptomatischen Therapie von Erkrankungen des rheumatischen Formenkreises eingesetzt. Dazu zählen u.a. die rheumatoide Arthritis, degenerative Formen wie Arthrosen und Spondylosen, sowie Polymyalgia rheumatica. Im wesentlichen unterscheidet man zwei Hauptwirkungen: Zum einen die analgetische und antipyretische und zum anderen die ausgesprochen antirheumatische/antiphlogistische Wirkung, die beide auf einer Hemmung der Prostaglandinsynthese beruhen. Somit läßt sich unter einer solchen Therapie die Lebensqualität der Patienten, allem voran die Schmerzfreiheit und die Erhaltung bzw. Verbesserung der Mobilität sowie der chronisch-progrediente Krankheitsverlauf günstig beeinflussen.

In neuerer Zeit gewinnen die pharmakologisch wirksamen NSAR aus der Gruppe der 2-Arylpropionsäurederivate als moderne Schmerz- und Rheumamittel zunehmend an Bedeutung. So besaß 1987 das Schmerzmittel Ibuprofen 2-(4-Isobutylphenyl)propionsäure oder $\alpha$-Methyl-(2-methylpropyl)benzolessigsäure, $C_{13}H_{18}O_2$ nur im rezeptfreien Bereich schon 13 % Anteil am gesamten Welt-Analgetika-Markt. Weiterhin stieg in Deutschland der Verbrauch an ibuprofenhaltigen Arzneimitteln nach der Entlassung aus der Rezeptpflicht deutlich an und man bezeichnet Ibuprofen bereits heute als das Schmerzmittel der 90er Jahre.

Die Dokumente EP-A-0 282 020 und WO-A-9 106 292 beschreiben Arzneimetteln, die Ibuprofen enthalten.

Neben Ibuprofen werden in den nächsten Jahren die analgetisch und antirheumatisch wirksamen Strukturverwandten, die alle ein asymmetrisches Kohlenstoffatom besitzen, wie z.B. Flurbiprofen, 2-(2-Fluor-4-biphenylyl)propionsäure $C_{15}H_{13}FO_2$ dessen Racemat in der antirheumatischen Therapie bereits deutlich niedriger (empfohlene Tagesdosis 150 - 200 mg) als das racemische Ibuprofen (bis zu 2400 mg täglich) dosiert wird, Ketoprofen, 2-(3-Benzoylphenyl)propionsäure $C_{16}H_{14}O_3$ und Tiaprofensäure 2-(5-Benzoyl-2-thienyl)propionsäure $C_{14}H_{12}O_3S$ in den Mittelpunkt des Interesses rücken. S-Naproxen, (S)-2-(6-Methoxy-2-naphthyl)propionsäure $C_{14}H_{14}O_2$, wird bereits heute erfolgreich als enantiomerreiner Wirkstoff aus dieser Substanzklasse in der Therapie eingesetzt.

Ein für den Galeniker schwieriges Problem dieser Wirkstoffgruppe ist in ihrer Schwerlöslichkeit zu sehen, denn nach der Theorie des passiven Transportes werden Wirkstoffe nur gelöst und undissoziiert resorbiert. Im allgemeinen wird versucht, durch Zerkleinern des Wirkstoffes (Mikronisieren) gemäß der Noyes-Whitney-Gleichung eine Vergrößerung der effektiven Stoffoberfläche A zu erreichen, was zur Erhöhung der Lösegeschwindigkeit führt. Dies hat wiederum eine Verbesserung der Bioverfügbarkeit zur Folge. Deshalb werden heute für oral zu applizierende schwerlösliche Analgetika/Antirheumatika bevorzugt mikronisierte Pulver eingesetzt.

Die Technik der Mikronisierung ist energie- und kostenaufwendig und es treten Probleme mit den sehr feinen Stäuben auf, wie z. B. die Gefahr einer Staubexplosion und die Gefahr der Staubinhalation durch das Personal, was umfangreiche Schutzmaßnahmen erfordert. Das Pulver selbst ist aufgrund elektrostatischer Aufladung bezüglich der Fließeigenschaften schwierig zu verarbeiten und ist meist schlecht benetzbar (Aerophilie). Infolge der hohen Produktionskosten liegen die Preise für mikronisiertes Pulver wesentlich höher als für herkömmliche Pulver.

Da sich derzeit jedoch keine Alternative bietet, wird diese Maßnahme ergriffen, um im Dünndarm, dem Resorptionsort für die 2-Arylpropionsäurederivate, eine ausreichende Lösungsgeschwindigkeit zu gewährleisten.

Bei den Wirkstoffen aus dieser Substanzklasse handelt es sich durchweg um schwache lipophile Säuren mit $pK_S$-Werten im Bereich von ca. 3 - 5. Dies bewirkt einen weiteren Nachteil, der darin besteht, daß gelöste oder solubilisierte Anteile bei Kontakt mit der sauren Magenflüssigkeit rekristallisieren können. Die entstehenden Kristalle können Magenirritationen hervorrufen. Da davon auszugehen ist, daß bei dem sauren Magen-pH-Wert von 1 das Lösungsgleichgewicht weitestgehend auf der Seite des ungelösten Wirkstoffes liegt, ist damit dieser Anteil der Wirkstoffdosis für eine Resorption im Magen verloren. Um eine solche "Inaktivierung" der Wirkstoffe im sauren pH-Bereich zu verhindern, werden Tabletten mit diesen Wirkstoffen üblicherweise mit magensaftresistenten Überzügen versehen. Solche Maßnahmen sind allerdings nicht unproblematisch. Zum einen muß die Lagerstabilität dieser Überzüge in Frage gestellt werden, wie beispielsweise der in vielen Fällen bereits nach einem Jahr Lagerung beschriebene Verlust an Magensaftresistenz zeigt.

Dies kann sich besonders nachteilig bei 2-Arylpropionsäurederivaten auswirken, da durch oben beschriebene Rekristallisationsvorgänge im Falle von mangelhaften magensaftresistenten Überzügen mit einer drastischen Einschränkung der Wirksamkeit sowie mit den beschriebenen Nebenwirkungen gerechnet werden muß.

Zum anderen weiß man heute aufgrund verbesserter Erkenntnisse über Magenphysiologie und Magenmotorik, daß Partikel ab einer Größe von 2 mm, also auch nicht zerfallende magensaftresistent überzogene Tabletten oder Dragees, eine längere Verweilzeit in Abhängigkeit vom Füllungszustand des Magens zeigen. Es ergeben sich Magenverweilzeiten von bis zu 10 Stunden. Damit ist der Zeitpunkt des Wirkungsanfangs kaum vorhersagbar und eine gezielte Therapie ist mit solchen Arzneiformen meist nicht möglich. Somit müssen diese galenischen Formen als nicht mehr zeitgemäß eingestuft werden. Strenggenommen darf man solche Arzneiformen deshalb nicht mehr als Akutformen bezeichnen, sie zeigen bereits "slow release".

Das Problem der Schwerlöslichkeit von 2-Arylpropionsäurederivaten wird im Stand der Technik weiterhin dadurch gelöst, daß man die Stoffe in gut wasserlösliche Salze überführt. Ibuprofen wird beispielsweise als wasserlösliches Lysinsalz angeboten, was im Vergleich zur freien Wirkstoffsäure einen schnelleren und signifikant höheren Blutspiegelmaximalwert $c_{max}$ bewirken soll. Handelsübliche Ibuprofen-Lysinat enthaltende Tabletten sind nicht magensaftresistent überzogen, sodaß das Auskristallisieren des Wirkstoffes im sauren Magen-Milieu mit allen oben genannten Nachteilen nicht verhindert werden kann. Daher ist auch keine schnellere Anflutung des Wirkstoffes in der Biophase unterhalb einer Stunde zu erwarten.

Weiterhin beschreibt Barabas in US Patent Nr. 4,704,436 die Erfindung eines wasserlöslichen Polyvinylpyrrolidon-Copolymer-Komplexes von Ibuprofen. Da als Copolymer z. B. Derivate von Ethylacrylaten oder Methacrylaten eingesetzt werden, muß man evtl. auftretende Nebenwirkungen durch solche synthetischen Polymere in Erwägung ziehen. Zwar ist der Komplex mit Ibuprofen wasserlöslich, es muß aber das Ibuprofen durch ein zeitlich vorgelagertes Gleichgewicht aus diesem Komplex wieder freigesetzt werden. Dies führt im hier beschriebenen Falle sogar zu so langen Auflösezeiten aus dem Komplex, daß er als geeignet für verzögerte Freisetzung bezeichnet wird.

Trotz geringerer gastrointestinaler Nebenwirkungen der Stoffklasse im Vergleich zu anderen Nichtsteroidalen Antirheumatika besitzen die 2-Arylpropionsäurederivate den großen Nachteil, daß die Resorption zeitverzögert erst im Dünndarm erfolgt.

Dies hat für ein Schmerzmittel, bei dem eine schnelle Wirkung erwünscht ist, einen späten Wirkungseintritt zur Folge. Im Falle von Ibuprofen besagt der pharmakokinetische Parameter $t_{max}$ von 1 - 2 Stunden, daß nach dieser Zeit das Blutspiegelmaximum erreicht ist. Es liegen Studien vor, wo bei zwei von 8 untersuchten Probanden therapeutische Konzentrationen erst nach mehr als 4 Stunden erreicht werden. In Kenntnis dieser Fakten wird es leicht verständlich, daß ein unter Schmerzen leidender Patient noch vor dem Wirkungseintritt der ersten Dosis eine zweite oder dritte Dosis einnimmt, weil der gewünschte analgetische Effekt vermeintlich ausbleibt. So setzt sich der Patient der Gefahr einer Überdosierung aus. Bei gefülltem Magen verlängern sich die Verweilzeiten zusätzlich, sodaß sich dieser Effekt hierdurch verstärken kann.

Betrachtet man zu diesem Faktor noch die Problematik der langen Magenverweildauer von magensaftresistent überzogenen Tabletten, so kann für $t_{max}$ im Falle der 2-Arylpropionsäurederivate ein noch größerer Wert resultieren und der Patient wird noch eher zu einer Mehrfacheinnahme verleitet.

J.J. Marty et al., Pharm. Acta Helv. 53, 1 (1978) S. 17-23 beschreibt die Herstellung von Gelatine-Nanopartikeln, in die auch Wirkstoffe eingeschlossen werden können. Bei der Herstellung dieser Gelatine-Nanopartikel wird zur Desolvatation und Resolvatation eine pH-Justierung vorgesehen. Eine Überführung des Arzneimittels in Nanopartikel wird nicht offenbart.

Der vorliegenden Erfindung liegt daher die Aufgabe zugrunde, Produkte sowie Verfahren zu ihrer Herstellung für die schnelle Freisetzung von 2-Arylpropionsäurederivaten, insbesondere Ibuprofen, Flurbiprofen, Ketoprofen, Tiaprofensäure, sowie Naproxen, zu entwickeln, die die oben zum Stand der Technik genannten Nachteile weitgehend vermeiden.

Diese Aufgabe wird erfindungsgemäß dadurch gelöst, daß das Arzneimittel das analgetisch und/oder antirheumatisch, sowie antipyretisch wirksame 2-Arylpropionsäurederivat in Form eines pharmazeutisch applizierbaren Nanosols und als Träger im wesentlichen Gelatine, ein Kollagenhydrolysat oder ein Gelatinederivat neben üblichen pharmazeutischen Hilfsstoffen enthält. Ein solches Nanosol weist eine innere Phase

4

aus dem 2-Arylpropionsäurederivat, das eine Teilchengröße von 10 - 800 nm, vorzugsweise unterhalb von 400 nm aufweist, und eine Oberflächenladung besitzt, eine äußere Phase aus Gelatine, Kollagenhydrolysat oder einem Gelatinederivat, welche(s) gegensinnig geladen ist, sowie einen annähernd oder vollständig isoionischen Ladungszustand der inneren und äußeren Phase auf.

Diese Aufgabe wird weiterhin durch ein Verfahren zur Herstellung eines kolloid-dispersen Systems eines 2-Arylpropionsäurederivates gelöst, das dadurch gekennzeichnet ist, daß man eine Gelatine nach ihrem isoelektrischen Punkt (IEP) so auswählt, daß ihr IEP mit dem Ladungszustand der Partikel des 2-Arylpropionsäurederivates so abgestimmt ist, daß die Gelatine bei einem bestimmten pH-Wert mit den ungelösten Partikeln des 2-Arylpropionsäurederivates zu Ladungs- neutralität führt; die Gelatine in die wäßrige Solform überführt; den pH-Wert in Abhängigkeit von dem IEP der Gelatine auf einen solchen Wert einstellt, daß die sich bildenden Nanopartikel des 2-Arylpropionsäurederivates annähernd oder vollständig ladungsneutral stabilisiert werden; und vor oder nach der letztgenannten Stufe das 2-Arylpropionsäurederivat in dem wäßrigen Gelatinesol löst oder eine Lösung des 2-Arylpropionsäurederivates mit dem wäßrigen Gelatinesol vereinigt.

Die Lösung der Aufgabe in obigem Sinne kann auch mit fraktionierter Gelatine erreicht werden.

Das 2-Arylpropionsäurederivat kann als Racemat, als racemisches Gemisch, als Pseudoracemat (Mischung von gleichen Anteilen von S- und R-Enantiomeren) oder Mischungen unterschiedlicher Anteile von S- und R-Enantiomeren im Bereich zwischen reinem S- und reinem R-Enantiomer vorliegen.

Ausführungsformen der erfindungsgemäßen Arzneimittel, sowie des Verfahrens zu ihrer Herstellung werden in den Unteransprüchen genannt und beansprucht.

In der internationalen (PCT-)Patentanmeldung vom heutigen Tage mit dem Titel "Pharmazeutisch applizierbares Nanosol und Verfahren zu seiner Herstellung" der ALFATEC-Pharma GmbH entsprechend der deutschen Patentanmeldung P 41 40 195.6 vom 5.12.1991, deren Inhalt auch zum Inhalt der vorliegenden Patentanmeldung gemacht wird, werden Nanosole und Verfahren zu ihrer Herstellung beschrieben, die es ermöglichen, kolloiddisperse Lösungen von in Wasser schwer löslichen Wirkstoffen durch Gelatine, Kollagenhydrolysate oder Gelatinederivate zu stabilisieren, wenn man den isoionischen Punkt (= Ladungsausgleich) zwischen Gelatine und den auf der Oberfläche geladenen Wirkstoffpartikeln wenigstens annähernd einstellt. Dabei bringt man das System Wirkstoffpartikel/Gelatine dadurch zum Ladungsausgleich, daß die Oberflächenladung der Partikel durch entsprechende Gegenladung der Gelatinemoleküle kompensiert wird. Erreicht wird dies durch Einstellung einer bestimmten Ladung auf den Gelatinemolekülen, die in Abhängigkeit zu ihrem isoelektrischen Punkt und dem pH-Wert der Lösung steht.

Fig. 1 zeigt eine schematische Darstellung der einstellbaren Ladungszustände von Gelatine in Abhängigkeit vom pH-Wert und IEP, wobei der IEP je nach Herstellungsart zwischen 3,5 und 9,5 liegen kann. Unterhalb von pH 3,5 sind fast alle Gelatinetypen positiv geladen. Im basischen Bereich oberhalb von pH 9,5 sind alle Gelatinetypen negativ geladen.

Fig. 2 zeigt den Mechanismus der passiven Arzneistoff-Resorption im Gastrointestinal-Trakt.

Erfindungsgemäß wird daher die Tatsache ausgenutzt, daß Gelatinen, Kollagenhydrolysate oder Gelatinederivate (nahezu unabhängig von der Viskosität) dann zu einem stabilen kolloid-dispersen Systems in Nanosolform führen, wenn der isoionische Ladungszustand zwischen Arzneistoffpartikel und Gelatine, Kollagenhydrolysat oder Gelatinederivat vorliegt.

Dagegen wurde Gelatine nach dem Stand der Technik nur zur Stabilisierung eines anorganischen, kolloid-dispersen Systems eingesetzt. So beschreibt z.B. das DAB 9 eine kolloidale Injektionslösung von radioaktivem Gold, die mit Gelatine hergestellt ist. Man stellte sich dabei lediglich vor, daß sich Makromolekül als "Kittsubstanz" zwischen den einzelnen Kolloidpartikeln befinde und so eine Teilchenaggregation verhindert werde.Dagegen war über den Stabilisierungsmechanismus, z.B. für Arzneistoffe, bisher nichts bekannt.

Die internationale (PCT-) Patentanmeldungen vom heutigen Tage der ALFATEC-Pharma GmbH und der PAZ Arzneimittelentwicklungsgesellschaft mbH entsprechend der genannten deutschen Patentanmeldung (vom 5.12.1991) betreffen die Akutform von S- und R-Ibuprofen (P 41 40 179.4), die Retardform von S- und R-Ibuprofen (P 41 40 172.7), die Akutform von S- und R-Flurbiprofen (P 41 40 184.0) und die Retardform von S- und R-Flurbiprofen (P 41 40 183.2). Ihre Offenbarung wird auch zum Gegenstand der vorliegenden Patentanmeldung gemacht.

Die Vorteile dieses neuartigen Produktes liegen auf der Hand. Durch eine kontrollierte Resorption der Wirkstoffe bereits im Magen kann die aufgrund ihrer Schwerlöslichkeit bisher als problematisch eingestufte Anflutungsgeschwindigkeit und Bioverfügbarkeit von 2-Arylpropionsäurederivaten erheblich verbessert werden.

Um die physiologischen Hintergründe der Resoprtion von Arzneistoffen im allgemeinen und die verbesserte Resorptionsquote der erfindungsgemäßen Nanosole ausreichend zu erläutern, ist zunächst eine Betrachtung zum Mechanismus der physiologischen Resorption von Arzneistoffen erforderlich, wie er auch in einschlägigen Publikationen dargestellt wird. Allerdings ist die vorliegende Erfindung weder an den folgenden Versuch einer wissenschaftlichen Erklärung der erfindungsgemäß auftretenden Phenomene gebunden noch kann sie hierdurch eingeschränkt werden.

Die passive Arzneistoffresorption erfolgt nach heutigem Erkenntnisstand (Theorie nach Brodie et al.), wenn folgende Bedingungen vorliegen:

a) die Gastrointestinalmembran wirkt als Lipidbarriere,

b) der Arzneistoff wird nur in gelöster und ungeladener, d.h. nichtionisierter Form aufgenommen,

c) saure Arzneistoffe werden bevorzugt im Magen, basische Arzneistoffe bevorzugt im Darm resorbiert.

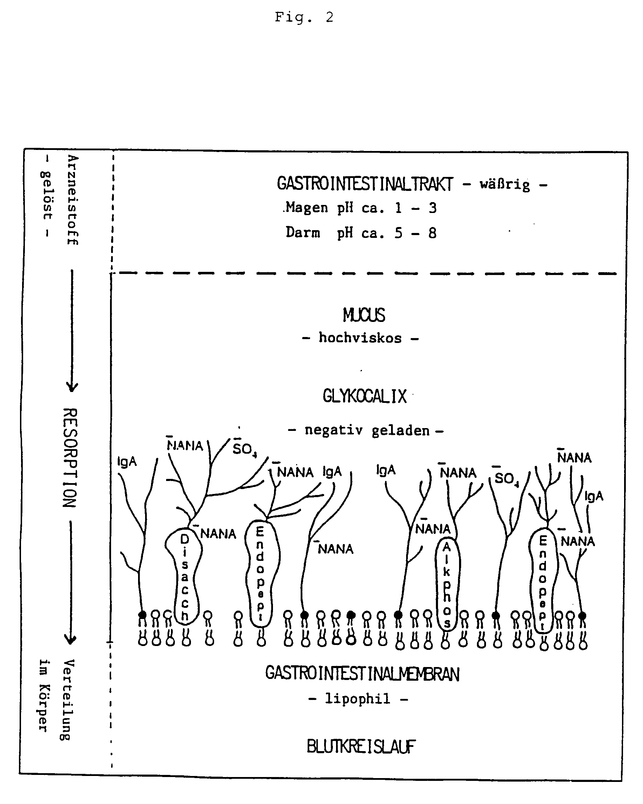

Nach der peroralen Aufnahme eines Arzneistoffs in den Organismus wird seine Resorption, d.h. der Übertritt in den allgemeinen Kreislauf (Biophase) in starkem Maße durch physikalische Barrieren behindert (siehe Abb. 2), nämlich

- durch die Mucus-Schicht und eine wässerige, daran adhärierende Schicht

- die Zellmembranen der intestinalen Epithelzellen mit der daran kovalent gebundenen Glykocalix sowie

- die sogenannten "Tight Junctions", die die Epithelzellen an ihrer apikalen Seite miteinander verbinden.

Diese Barrieren bedingen, daß die Resorption von Arzneistoffen hauptsächlich abhängig von ihrem Verteilungsmechanismus und Ladungszustand- durch die Lipid-Doppelschichten erfolgt (sogenannte passive Diffusion).

Die Epithelzellen des gesamten Magen-Darm-Traktes sind mit einer Mucus-Schicht bedeckt, die aus Mucinen (Glykoproteinen), Elektrolyten, Proteinen und Nucleinsäuren besteht. Vor allem die Glykoproteine bilden mit dem Hauptanteil des Mucus, nämlich Wasser, eine viskose Gelstruktur, die in erster Linie Schutzfunktionen für die darunter liegende Epithelschicht ausübt. Die Mucusschicht ist an die apikale Oberfläche der Epithelzellen über die Glykocalix gebunden. Die Glykocalix hat ebenfalls eine Glykoproteinstruktur, die kovalent an Bausteine der Membran-Doppelschicht der Epithelzellen gebunden ist. Die verzweigten Polysaccharide der Glykocalix, die entweder direkt an amphiphile Moleküle der Doppelmembran oder an die Doppelmembran inkorporierte Proteine kovalent gebunden sind, besitzen geladene N-Acetyl-Neuraminsäure- und Sulfat-Reste und sind daher negativ geladen, was zu einer elektrostatischen Bindung oder Abstoßung von geladenen Arzneistoffmolekülen bzw. von elektrostatisch geladenen Partikeln führen kann. Die Epithelzellmembranen bestehen aus Phospholipid-Doppelschichten, in die Proteine über ihre hydrophoben Bereiche verankert sind. Die Phospholipid-Doppelschichten mit ihrem lipophilen Anteil stellen eine weitere Barriere für den Transport der zu resorbierenden Arzneistoffe dar.

Aus dieser Darstellung geht deutlich hervor, daß geladene Arzneistoffmoleküle bzw. elektrostatisch geladene Partikel daher nur eine sehr geringe Chance haben, über den peroralen Applikationsweg resorbiert zu werden.

Die erfindungsgemäßen Nanosole geben erstmalig die technische Lehre, ein System zu bilden, mit dem diese vorgenannten Resorptionshindernisse zu überwinden sind. Da die Wirkstoff-Nanopartikel durch die Gelatine erfindungsgemäß ladungsneutral stabilisiert werden, kann ihr Transport durch die negativ geladene Glykocalix ohne größere Hindernisse erfolgen, im Gegensatz zu sonstig beschriebenen Nanopartikeln des Standes der Technik, die nicht ladungsneutral stabilisiert werden bzw. stabilisiert werden können. Erfindungsgemäß kann die Einstellung des isoionischen Ladungszustandes zusätzlich noch in Abstimmung auf die physiologischen Verhältnisse erfolgen (siehe insbesondere die Ausführungen auf S. 9, Zeile 14-20 in Verbindung mit Beispiel 1 und 2).

Da die erfindungsgemäßen Wirkstoff-Nanosole die Glykocalix ungehindert passieren können, ohne durch elektrostatische Effekte gebunden bzw. abgestoßen zu werden, erreichen sie damit auch die Oberfläche der Epithelzellen und stehen dort in hoher Konzentration zur Verfügung.

Nun können auch aktive, carriervermittelte Transportmechanismen bzw. Phagozytose einen wesentlichen Beitrag zur Resorption der Wirkstoff-Nanosole liefern.

Grundsätzlich eignen sich für die Herstellung einer Akutform alle Gelatinequalitäten, sofern sie nach Weiterverarbeitung zur Arzneiform in ihrem Auflösungsverhalten nicht eingeschränkt werden. Beispielsweise lösen sich sprüh- oder gefriergetrocknete Nanosol-Pulver oberhalb 37°C in wenigen Minuten vollständig auf.

Stellt die spätere Arzneiform ein Komprimat dar, kann man durch Auswahl von Gelatinesorten im Molekulargewichtsbereich unterhalb $10^5$ D, vorzugsweise im Bereich von $10^4$ - $9,5 \times 10^4$ D schnelle Freisetzungszeiten, gegebenenfalls unter Zusatz geeigneter Hilfsstoffe erzielen. Gelatinesorten mit Peptidanteilen >80% lassen sich technologisch vorteilhafterweise direkt verpressen.

In Abhängigkeit von der Herstellungsweise von Gelatine (Ausmaß des Abbaus des nativen Kollagens und saures bzw. alkalisches Aufschlußverfahren) weist Gelatine vom Typ A oder Typ B ein charakteristisches Molekulargewichtsspektrum bzw. Molekulargewichtsverteilung auf. In Tabelle 1 sind die Molekulargewichtsverteilungen von verschiedenen Gelatinetypen bzw. von Kollagenhydrolysaten angegeben, sowie der prozentuale Anteil (Häufigkeit) einzelner Molekulargewichtsbereiche.

Tabelle 1

| Molekulargewichtsverteilung von verschiedenen bekannten Gelatinetypen bzw. von bekannten Kollagenhydrolysaten | | | | | | | |
|---|---|---|---|---|---|---|---|
| Molecular Mass Distribution (kD) | Native Collagen % | Gelatin Type B % | Gelatin Type A % | Collagen hydrolysate Gelita® Collagel A | Collagen hydrolysate Gelita® Collagel B | Collagen hydrolysate Gelita® Sol C | Elastin hydrolysate Gelita® Gelastin |
| >360 | 100 | 18,0 | 18,0 | 0 | 0 | 0 | 0 |
| 285 | 0 | 7,0 | 9,0 | 0 | 0 | 0 | 0 |
| 145-237 | 0 | 20,0 | 34,0 | 1,0 | 1,5 | 0 | 0 |
| 95 | 0 | 26,0 | 11,0 | 0 | 0 | 0 | 0 |
| 95-50 | 0 | 16,3 | 13,4 | 2,6 | 4,0 | 1,1 | 0 |
| 50-20 | 0 | 7,4 | 9,1 | 18,0 | 14,5 | 0,3 | 0 |
| 20-10 | 0 | 3,9 | 3,8 | 43,0 | 31,5 | 3,7 | 0,2 |
| 10-5 | 0 | 3,0 | 3,0 | 15,4 | 20,0 | 12,2 | 5,2 |
| 5-2 | 0 | 0 | 0 | 6,0 | 14,0 | 26,0 | 93,9 |
| 2-1 | 0 | 0 | 0 | 7,0 | 8,0 | 23,0 | 0 |
| <1 | 0 | 0 | 0 | 6,5 | 7,0 | 34,0 | 0 |
| MG | 360 | 165 | 185 | 12-18 | 12-18 | 3 | 2-3 |

Man erkennt in den einzelnen Spalten deutlich das Überwiegen eines einzelnen Bereiches im Vergleich zu den übrigen Molekulargewichtsbereichen derselben Gelatine. Dieser Bereich stellt also das Maximum der Molekulargewichtsverteilung dar (es liegt z.B. bei der in der Abbildung aufgeführten Gelatine Typ B bei 95 kD). Der Begriff des "Maximums der Molekulargewichtsverteilung" ist jedoch streng zu trennen von dem Begriff des "durchschnittlichen mittleren Molekulargewichts". Dieser Mittelwert liegt bei der erwähnten Gelatine vom Typ B bei 165 kD.

Die erfindungsgemäßen Nanosole zeigen in vitro bei pH 1-4 kein Teilchenwachstum innerhalb von 20 Stunden und mehr, d.h. es erfolgt keine Flockung oder Auskristallisation. Das bedeutet, daß das Nanosol ausreichend lang während der Magenverweilzeit und unabhängig von auftretenden pH-Schwankungen, z.B. durch Nahrungseinfluß und zudem noch unabhängig vom Füllungszustand des Magens der Magenmucosa für die Resorption zur Verfügung steht.

Es lassen sich erfindungsgemäß auch Nanosole der 2-Arylpropionsäurederivate herstellen, die bzgl. ihres isoionischen Ladungszustandes auf die physiologischen Verhältnisse direkt abgestimmt werden. So kann man, wie aus Beispiel 2 hervorgeht, unter gleichen Herstellungen wie in Beispiel 1 allein durch entsprechende Wahl des IEP's der eingesetzten Gelatine das Stabilitätsmaximum im sauren Bereich erhöhen.

Die Teilchen der Nanosole liegen nach ihrer Herstellung, nach Resuspendierung des getrockneten Pulvers und nach Resuspendierung aus einer Arzneiform in Teilchengrößen von 10 bis 800 um und bevorzugt unterhalb 400 um nahezu monodispers vor.

Das Nanosol wird in Verbindung mit einer schnell freisetzenden Akutarzneiform als Nanodispersion im Magen gut verteilt, was optimale Voraussetzungen für die Resorption schafft. Erstaunlicherweise liegen die Nanopartikel der 2-Arylpropionsäurederivate auch unter physiologischen Bedingungen stabilisiert vor und können als solche besonders schnell resorbiert werden, ohne daß sie vorher aufgelöst werden müssen. Damit entfallen zeitlich vorgelagerte Lösungsgleichgewichte, wie sie allgemein bekannt und im Stand der Technik beschrieben sind in jedem Fall. Sie verhalten sich demnach in der pharmazeutischen Anwendung nahezu wie echte Lösungen, ohne aber eine solche zu sein.

Somit wird erstmals durch die vorliegende Erfindung eine kontrollierte Resorption im Gastrointestinaltrakt bereits während der Magenverweilzeit möglich. Die Resorption ist nicht mehr pH-abhängig auf den Dünndarmbereich beschränkt und es wird eine schnelle Anflutung für 2-Arylpropionsäurederivate mit entsprechend schnellem Wirkeintritt erzielt.

7

Damit ist es möglich, bei diesen Arzneistoffen erstmals einen $t_{max}$-Wert unterhalb von 2 h, insbesondere unterhalb von 1 h zu erreichen.

Überraschenderweise läßt sich auch eine Erhöhung des Blutspiegelmaximalwertes $c_{max}$ feststellen. Die Erhöhung von $c_{max}$ kann daher unter Umständen eine Dosisreduktion bei gleicher Wirksamkeit zur Folge haben.

Die schnelle Anflutung führt neben dem schnellen Wirkungseintritt zu einer früheren Elimination aus dem Plasma, sodaß die systemische Belastung gegenüber herkömmlichen Arzneimitteln verkürzt wird. Die Wirkdauer selbst wird dadurch praktisch nicht verkürzt, weil am Wirkort, insbesondere bei entzündlichen Prozessen, mit einer wesentlich längeren Verweildauer des Wirkstoffs gerechnet werden kann. Die Halbwertszeiten im Plasma betragen im Falle von Ibuprofen beispielsweise ca. 2 h. In der Synovialflüssigkeit wurden dagegen 10 h bis 12 h gefunden.

Wie in-vitro Versuche gezeigt haben, kann aufgrund der langen Stabilitäten der erfindungsgemäßen Nanosole keine Rekristallisation im Magen stattfinden.

Hierin liegt ein weiterer wesentlicher Vorteil der vorliegenden Erfindung: Es ist bekannt, daß gerade bei älteren Patienten mit der häufigsten Inzidenz rheumatischer Erkrankungen auch die Schwere der wirkstoffbedingten Nebenwirkungen zunimmt. Dies gilt im besonderem Maße für die Dauertherapie der chronisch Erkrankten. Da in einem 2-Arylpropionsäurederivat-Nanosol nun der Wirkstoff quasi in Gelatine "eingebettet" vorliegt und nicht auskristallisieren kann, da er nicht gelöst vorliegt, ist auch ein Schleimhautschutz im Gastrointestinaltrakt durch das untoxische Biopolymer Gelatine zu erwarten.

Bei der Formulierung von Akut- bzw. Retardpräparaten macht der Pharmazeut einen grundsätzlichen Unterschied zwischen:

1. galenischer Zubereitung, d.h. einer Freisetzung des Arzneistoffes, z.B. aus einer Tablette in zeitlich schneller (Akutform) oder verlangsamter (Retardform) Weise;
und

2. dem arzneistoffspezifischen Resorptionsort, wie z.B. Magen oder bestimmte Darmabschnitte.

Die erfindungsgemäßen Nanosole sind in der Lage, unabhängig von der galenischen Zubereitung, aufgrund ihrer speziellen Zusammensetzung, im gesamten gastrointestinalen Bereich resorbiert zu werden. Sie können daher vorteilhaft zu Akut- bzw. Retardarzneiformen weiterverarbeitet werden.

Die im Rahmen dieser Erfindung beanspruchten Akut-Nanosole lassen sich mit Retard-Nanosolen auf neuartige Weise kombinieren. Setzt man beispielsweise hochmolekulare Gelatinesorten ein, so lassen sich Nanosole mit Retardwirkung ezielen. Als besondere Ausführungsform für diese Retard-Nanosole ist z.B. eine Matrixtablette geeignet, wie sie in der internationalen (PCT-)-Patentanmeldung mit dem Titel "Solgesteuerte Thermokolloidmatrix auf Gelatinebasis für perorale Retardformen" (11 AL2713) der ALFATEC-Pharma GmbH vom selben Tag, die der deutschen Patentanmeldung P 41 40 192.1 entspricht, beschrieben wird. Ihre Offenbarung wird auch zum Gegenstand der vorliegenden Patentanmeldung gemacht.

Sinnvolle Kombinationen können z.B. sein: Akut S-Ibuprofen mit Retard S-Ibuprofen, Akut R-Flurbiprofen (analgetisch) mit Retard S-Flurbiprofen (antiphlogistisch) und weitere.

Das getrocknete Nanosol kann zu Arzneiformen, beispielsweise zu einer Tablette, weiterverarbeitet und daraus resuspendiert werden. Somit wird ein magensaftresistenter Überzug zum Schutz vor "Inaktivierung" des Wirkstoffes durch den sauren Magen-pH überflüssig.

Die Gefahr einer Überdosierung durch Mehrfacheinnahme wird durch den schnellen Eintritt der Analgesie als Folge der Resorption im Magen ausgeschlossen. Alle genannten Nachteile und Gefahren des magensaftresistenten Überzugs entfallen. Somit dient die vorliegende Erfindung auch der Erhöhung der Patienten-Compliance. Dies alles stellt einen entscheidenden Beitrag zur geforderten Arzneimittelsicherheit dar.

Grundsätzlich läßt sich das erfindungsgemäße Produkt zu allen peroral zu applizierenden Arzneiformen verarbeiten, insbesondere kann es direkt als Pulver in Hartgelatinekapseln abgefüllt werden. Es eignet sich auch hervorragend zur Direkttablettierung. Eine Verarbeitung zu einem Trinkgranulat, schnellauflösenden Pellets oder Trinktabletten ist für die Applikation als schnellanflutende Akutform von besonderem Interesse.

Des weiteren sind die erfindungsgemäßen Nanosole auch für die Verarbeitung in Salben-, Creme- oder Gelgrundlagen für den topischen Anwendungsbereich (Rheumasalben) einsetzbar.

Prinzipiell sind zur Herstellung der erfindungsgemäßen Nanosole die in der o.g. deutschen Patentanmeldung P 41 40 195.6 der ALFATEC-Pharma GmbH "Pharmazeutisch applizierbares Nanosol und Verfahren zu seiner Herstellung" genannten Vorgehensweisen und Verfahrensvarianten geeignet, die im folgenden noch einmal angeführt werden:

Es werden mehrere Verfahren zur Herstellung der Nanosole vorgeschlagen. Dabei handelt es sich um eine beispielhafte, unvollständige Aufzählung.

## Verfahren I

Dieses kann angewendet werden, wenn der Arzneistoff in einer Mischung aus: einem mit Wasser mischbaren organischen Lösungsmittel und Wasser, oder aus mehreren mit Wasser mischbaren organischen Lösungsmitteln und Wasser löslich ist:

a) eine in den Vorversuchen ausgewählte Gelatine wird mit Wasser in Solform überführt;

b) der in den Vorversuchen gefundene pH-Wert der Lösung wird eingestellt;

c) ein oder mehrere mit Wasser mischbare(s), organische(s) Lösungsmittel, vorzugsweise Ethanol, Isopropanol oder Methanol, wird/werden zu dieser Lösung gegeben;

d) der Arzneistoff wird in fester Form zu der Lösung gegeben und gelöst;

e) das/die organische(n) Lösungsmittel wird/werden entfernt, vorzugsweise durch Eindampfen in Vakuum; dabei entsteht das Nanosol;

f) die kolloid-disperse Lösung wird anschließend, vorzugsweise durch Sprüh- oder Gefriertrocknung, getrocknet.

Das organische Lösungsmittel hat die Aufgabe, den Arzneistoff zu lösen und verändert auch die Hydrathülle der Gelatinemoleküle.

## Verfahren II

Diese Ausführungsform kann angewendet werden, wenn der Arzneistoff eine Säure oder eine Base ist, deren Salz in Wasser löslich ist:

a) eine in den Vorversuchen ausgewählte Gelatine wird mit $H_2O$ in die Solform überführt;

b) es wird ein solcher pH-Wert eingestellt, der die Salzbildung des Arzneistoffs ermöglicht;

c) der Arzneistoff wird unter Salzbildung in dem Gelatinesol gelöst;

d) durch Zugabe von Alkohol oder ähnlichen organischen Lösungsmitteln kann die Hydrathülle der Gelatinemoleküle gelockert werden;

e) durch Zugabe einer geeigneten Menge Säure oder Base wird der pH-Wert eingestellt, der zur Bildung des isoionischen Punkts (IIP) führt, dabei entsteht das Nanosol;

f) die kolloid-disperse Lösung wird wie in Verfahren I getrocknet.

Stufe d) ist fakultativ, jedoch bevorzugt.

## Verfahren III

Diese Ausführungsform kann angewendet werden, wenn der Arzneistoff ein Neutralstoff ist:

a) es wird ein Gelatinesol hergestellt, wie unter (1) a) und b) beschrieben.

b) eine zweite Lösung aus einem mit Wasser mischbaren organischen Lösungsmittel, vorzugsweise Ethanol, Methanol, Isopropanol, Aceton und dem Arzneistoff wird hergestellt.

c) die beiden Lösungen werden vereinigt.

d) das organische Lösungsmittel wird entfernt und die kolloid-disperse Lösung wird getrocknet.

## Verfahren IV

a) Wie unter (I) a) und b) beschrieben.

b) In einer zweiten Lösung wird ein kolloid-disperses System mit dem Arzneistoff kurzzeitig gebildet, jedoch ohne Gelatine.

c) Die unter (b) erhaltene Lösung wird kontinuierlich mit der Gelatinelösung vereinigt.

Bei Schritt (IV) c) kann die kontinuierliche Vermischung der unter (IV) a) und b) beschriebenen Lösungen zeitabhängig durch on-line Messung der Teilchengröße mit einem geeigneten Verfahren, wie z.B. durch Laser-Licht-Streuung (BI-FOQELS On-line Particle Sizer), gesteuert werden. Damit ist es möglich, eine gewünschte Partikelgröße kontinuierlich einzustellen.

Alle genannten Verfahren sind auch für Kollagenhydrolysate und Gelatinederivate geeignet und können problemlos in den technischen Maßstab übertragen werden.

Die wesentlichen Schritte können weitgehend automatisiert ablaufen, wobei auch Verfahren I bis III kontinuierlich durchführbar sind. Im Falle der Akutform für 2-Arylpropionsäurederivate seien als bevorzugt geeignete Verfahren die Varianten Nr. II und III genannt.

Für die erfindungsgemäßen Akutformen eignen sich alle Gelatinen, Gelatinederivate, Kollagenhydrolysate und fraktionierte Gelatine, sowie deren Mischungen. Gelatinesorten, die einen erfindungsgemäß beschriebenen isoelektrischen Punkt (IEP) aufweisen, der nicht handelsüblich ist, können nach den Beispielen I bis III aus o.g. deutscher Patentanmeldung hergestellt werden.

Gegenüber handelsüblichen Produkten führt die Verwendung von Gelatine, die auf spezielle Weise hergestellt wurde, zu erfindungsgemäß beschriebenen Nanosolen mit erhöhter Stabilität.

Beispiele für die Herstellung erfindungsgemäß besonders geeigneter Gelatinequalitäten werden unten gegeben.

**Beispiele für die Herstellung von erfindungsgemäß besonders geeigneten Gelatinesorten mit isoelektrischen Punkten von 3,5 bis 9,5**

Beispiel I:

**Verfahren zur Erzielung eines IEP's von 7,5 bis 9,5**

Kollagenhaltiges Ausgangsmaterial wie z.B. Schweineschwarten werden mit einer wäßrigen Lösung einer 0,45 N Mineralsäure, vorzugsweise Schwefelsäure, im Flottenverhältnis 1:1 12 bis 20 Stunden behandelt. Anschließend wird der Säureüberschuß durch mehrmaliges Waschen entfernt, wobei zur Abkürzung des Verfahrens Natriumhydrogencarbonat verwendet werden kann. Die Extraktion des sudreifen Materials erfolgt mit heißem Wasser bei 55 - 80° C bei einem pH von 2,5 bis 4,5. Bei pH-Werten unterhalb von 3,5 kann ein IEP von 8,5 bis 9,5 erreicht werden, bei pH-Werten oberhalb 3,5 liegt der IEP bei 7 bis 8,5. Auf diese Weise lassen sich verschiedene IEP's von 7 bis 9,5 in direkter Abhängigkeit vom pH-Wert während der Extraktion erzielen.

Nach der Verfahrensstufe der Extraktion wird die wäßrige Lösung neutralisiert und wie üblich aufgearbeitet.

Durch dieses Verfahren kann man weiterhin in Abhängigkeit von der gewählten Temperatur während der Extraktion Gelatinesorten mit hohen bis mittleren Molekulargewichtsverteilungen erhalten.

Bei Temperaturen von 50-55° C erhält man besonders hochviskose und hochbloomige Qualitäten. Gelatinesorten mit niedrigem Molekulargewicht bzw. kaltwasserlösliche Gelatinen können durch gezielten Abbau mit Kollagenasen erhalten werden.

Beispiel II:

**Verfahren zur Erzielung eines IEP's von 4 bis 7,5**

Das kollagenhaltige Ausgangsmaterial wird zur Entfernung von Fremdstoffen zunächst gewaschen, zerkleinert und anschließend durch Zusatz von Magnesit, Natronlauge oder Calciumhydroxid durch gründliches Vermischen im Flottenverhältnis 1:1,2 homogen alkalisch gemacht. Das so vorbehandelte Material wird kurzzeitig druckhydrolytisch bei $1,01 \times 10^5$ bis $2,02 \times 10^5$ Pa und einem pH-Wert der wäßrigen Lösung von 8-14 aufgeschlossen. Nach dem Aufschluß wird sofort neutralisiert und die noch heiße wäßrige Gelatinelösung wie üblich filtriert, entsalzt, aufkonzentriert und getrocknet.

Nimmt man ein schwach basisches Aufschlußmittel wie Magnesit, erhält man einen IEP von 6 bis 7,5, sofern man bei $1,01 \times 10^5$ Pa arbeitet. IEP's von 5 bis 6 erhält man bei Einsatz einer verdünnten Kalkmilchsuspension und bei Verwendung von 0,005 bis 0,1 N Natronlauge können IEP's von 4 bis 5 erzielt werden.

Gelatinesorten mit geringem Racemisierungsgrad und niedrigem Peptidanteil lassen sich bei Druckverhältnissen von $1,01 \times 10^5$ Pa und Verweilzeiten von maximal 10 Min. erreichen.

Mittel- bis niedrigmolekulare bis hin zu kaltwasserlöslichen Sorten ergeben sich durch entsprechend längere Verweilzeiten.

Beispiel III:

**Verfahren zur Erzielung eines IEP's von 3,5 bis 6**

Kollagenhaltiges Ausgangsmaterial, vorzugsweise Spalt bzw. Ossein, wird nach der Eingangswäsche einem Kurzzeitäscher unterworfen. Hierbei bieten sich zwei Verfahrensvarianten im Flottenverhältnis 1:1,3 an, die entweder eine gesättigte Kalkmilchsuspension oder eine 0,1 bis 1 N Natronlauge zum Einsatz bringen.

Bei Verwendung einer Kalkmilchsuspension wird das Rohmaterial unter ständiger Bewegung maximal 3 bis 4 Wochen aufgeschlossen. Anschließend wird das Material durch Säurezugabe neutralisiert und mehrmals gewaschen. Die weitere Aufarbeitung folgt wie üblich. Auf diese Weise lassen sich IEP's von 4 bis 6 einstellen.

Bei Einsatz von Natronlauge läßt sich der Äscherprozeß nochmals verkürzen, wobei bei Konzentrationen von 1 N Natronlauge das Material je nach Zerkleinerungsgrad bereits nach 6 - 12 Stunden aufgeschlossen ist. Die Neutralisation erfolgt mit äquimolaren Mengen Mineralsäure und die Neutralsalze werden durch mehrmaliges Waschen oder durch Entsalzen der in der Extraktion gewonnenen wäßrigen Gelatinelösung entfernt. Bei dieser Verfahrensvariante lassen sich IEP's von 3,5 bis 5 erhalten.

Besonders peptidarme Gelatinesorten werden bei kurzer Verweilzeit im Äscher erhalten. Man kann so Gelatinesorten mit hoher bis mittlerer Molekulargewichtsverteilung ($M = 10^4 - 10^7$ D) erhalten.

Niedrigmolekulare bis kaltwasserlösliche Gelatinesorten kann man durch thermischen Abbau bzw. enzymatisch erhalten.

Bevorzugt werden im Falle der 2-Arylpropionsäurederivate Gelatinesorten mit IEP von 3,5 bis 9,5 eingesetzt.

Übliche pharmazeutische Hilfsstoffe und/oder weitere Makromoleküle können, sofern sie technologisch erforderlich sind, in flüssigem oder getrocknetem Zustand den erfindungsgemäßen Nanosolen zugesetzt werden.

Zum Beispiel kann ein Zusatz von Polyvinylpyrrolidon im Mengenverhältnis Gelatine zu Polyvinylpyrrolidon im Bereich von 5:1 bis 500:1 geeignet sein.

Eine Akutform im Sinne der Erfindung, die z.B. zu Tabletten verarbeitet wird oder lyophilisiert werden soll, kann durch Zusatz von niedrigmolekularen Polyvinylpyrrolidonsorten im Bereich von 10:1 bis 50:1 in den technologischen Verarbeitungseigenschaften verbessert werden, ohne daß die Stabilität der Nanosole negativ beeinflußt wird.

Die in den folgenden Beispielen bevorzugten Herstellungsverfahren, Vorgehensweisen und Bezeichnungen beziehen sich wie folgt auf die deutschen Patentanmeldungen "Pharmazeutisch applizierbares Nanosol und Verfahren zu seiner Herstellung" (P 41 40 195.6) bzw. die oben genannten Verfahren und Beispiele:

Nanosol-Herstellung: Verfahren II und III

Gelatineherstellung: Beispiel I bis III

Vortest: siehe folgende Beschreibung:

Vortest:

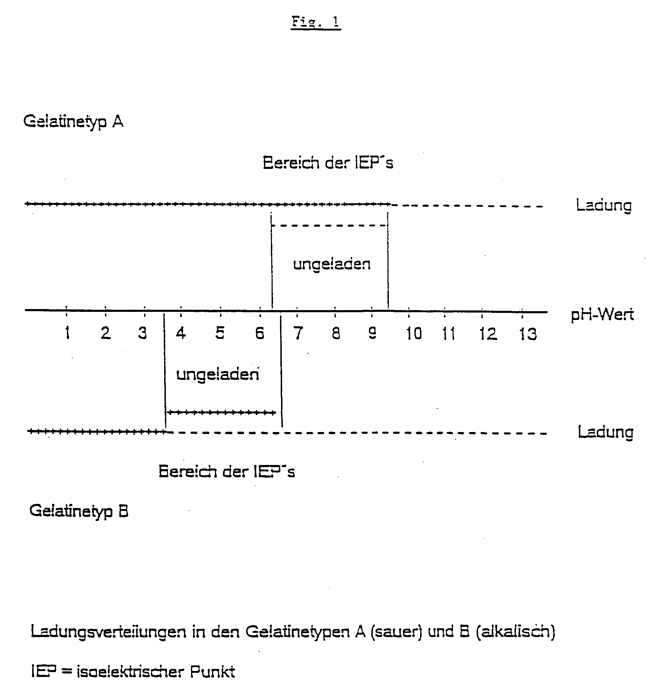

Wie eingangs schon erwähnt und wie aus Fig.1 ersichtlich ist, hängt die absolute, maximal mögliche Nettoladung eines einzelnen Gelatinemoleküls hauptsächlich von der Anzahl der freien COOH- und $NH_2$-Gruppen und dem pH-Wert der Lösung ab. Da sich Typ A, B, Kollagenhydrolysate oder Gelatinederivate in der Anzahl freier COOH-Gruppen unterscheiden, ist damit auch ihre maximal mögliche Nettoladung unterschiedlich. Bei Gelatinederivaten kann der Ladungszustand zusätzlich von der Art der Modifizierung abhängen.

Bei der Durchführung des erfindungsgemäßen Verfahrens wählt man in einem Vortest die geeignete Gelatine und den geeigneten pH-Wert aus.

Zunächst wird ein den physikalisch-chemischen Eigenschaften des Arzneistoffs angepaßter Arbeits-pH-Bereich gewählt. Als physikalisch-chemische Eigenschaft des Arzneistoffs sind vor allem zu berücksichtigen: Die Löslichkeit (in organischen Lösungsmitteln bzw. Wasser), seine Eigenschaft als Säure, Base oder Neutralstoff sowie seine Stabilität gegenüber Säuren und Laugen.

In einem ersten Schnelltest wird festgestellt, welche Ladung die ausgefällten Partikel besitzen. Daraus ergibt sich, unter Berücksichtigung des Arbeits-pH-Bereichs, die Wahl eines geeigneten Gelatinetyps. Sind die Teilchen beispielsweise negativ geladen, sucht man eine Gelatine aus, die unter den gegebenen pH-Bedingungen positiv geladen ist. Dieser Schnelltest zur Feststellung der Partikelladung hat die Vorteile, daß er ohne großen apparativen und zeitlichen Aufwand durchgeführt werden kann. So kann auf eine zeitaufwendige und ungenaue Zeta-Potential-Messung gänzlich verzichtet werden.

In vielen Fällen wird es ausreichend sein, für diesen Schnelltest zwei handelsübliche Gelatinen Typ A und B mit einem IEP von 9,5 bzw. 3,5 mit Peptidanteilen <30 % und einer Bloomzahl von 200, die weiterhin als Standardgelatinen bezeichnet werden, bei einem pH-Wert von 6 in die Solform zu überführen (5%ige wäßrige Lösung) und den Arzneistoff in einem mit Wasser mischbaren Lösungsmittel, wie z. B. Ethanol, Isopropanol oder Aceton, zu lösen und jeweils mit den Gelatinelösungen homogen zu mischen. Bei gleicher Dosierung des Arzneistoffs wird sich bei der in ihrem Ladungszustand nicht geeigneten Gelatine ein kolloidales System entweder nicht ausbilden oder sofort instabil werden bzw. der Arzneistoff ausflocken.

Sind die entstehenden Partikel negativ geladen, werden sie eher von Gelatinelösung mit Typ A, der bei einem pH-Wert von 6 positiv geladen ist, stabilisiert als von der Lösung mit Gelatine Typ B; im Gegenteil wird in diesem Fall Typ B entweder kein kolloidales System ausbilden oder das System sofort destabilisieren. Das Ausflocken der Teilchen läßt sich z. B. über eine einfache Trübungs-Messung verfolgen.

Bei diesem Schnelltest muß auf jeden Fall der Arbeits-pH-Bereich beachtet werden. Man kann auch andere Gelatinen als Standard auswählen, sie müssen jedoch in ihrem IEP so gewählt werden, daß sie bei diesem pH-Wert entgegengesetzte Nettoladung tragen (siehe auch Fig.1). In den meisten Fällen werden die besagten Standardgelatinen Typ A und B für diesen Schnelltest ausreichen.

Ausgehend vom Ergebnis des Vorversuchs werden nun durch schrittweise Variation des IEP's durch Verwendung entsprechender Gelatinesorten und des pH-Wertes der Lösung in kleineren Bereichen (z. B. 0,1 pH-Schritte) die optimalen Bedingungen zur Bildung der Nanosole ermittelt. D.h. es muß das Stabilitätsoptimum, das durch den isoionischen Punkt (IIP) gekennzeichnet ist, gefunden werden, um eine ausreichende Stabilität für die genannten pharmazeutischen Anwendungen zu gewährleisten.

Es kann durchaus der Fall sein, daß eine im Sinne der Erfindung akzeptable Stabilität der Nanosole bereits in einem engeren pH-Bereich (ca. 0,5 Einheiten) um den isoionischen Punkt gefunden wird, so daß eine Einstellung dieses Punktes selbst nicht unbedingt notwendig ist. Andererseits können auch mehrere Gelatinen zu den gleichen, stabilen Ergebnissen führen. So kann beispielsweise (Beispiel 5) mit dem oralen Antidiabetikum Glibenclamid bei einem Gelatinetyp B mit einem IEP von 5,5 das Stabilitätsoptimum bei einem pH-Wert von 3,2 liegen, während bei einem Gelatinetyp B mit einem IEP von 3,8 das Stabilitätsoptimum bei einem pH-Wert von 2,2 liegt.

Gekennzeichnet durch ein Stabilitätsmaximum, wurde in beiden Fällen der isoionische Punkt erreicht (die Abhängigkeit der Nettoladung vom pH-Wert und dem IEP muß nicht linear sein, da sie durch den $pK_S$-Wert der vorhandenen COOH- bzw. $NH_3^+$-Gruppen gegeben ist).

Die folgenden Beispiele sollen die Erfindung näher erläutern:

**Beispiel 1**

Arzneistoff: Ibuprofen (Racemat), Wirkstoffsäure
Gelatinetyp: handelsüblich, Typ B, 40 Bloom
Nanosol-Herstellung: analog Verfahren II
Gewichtsverhältnis Gelatine/Arzneistoff: 2 : 1
Der Arbeits-pH-Bereich für Ibuprofen liegt unterhalb seines $pK_S$-Werts von 4,6
Bei einem pH-Wert von 3,0 weist eine Gelatine Typ B (IEP 4,9) ein Stabilitätsmaximum auf.

10 kg einer 6%igen wäßrigen Gelatinelösung werden mit destilliertem Wasser (40°C) hergestellt.

Unter Rühren werden 300 g racemisches Ibuprofen in 800 g Natronlauge (10%) gelöst und mit der Gelatinelösung vereinigt. Es wird bei 40°C solange weitergerührt, bis eine völlig klare Lösung entsteht. Danach wird durch Zugabe von Salzsäure auf pH 3,0 eingestellt, wobei sich das Nanosol bildet.

Nach Aufkonzentrierung und Sprühtrocknung werden aus dem erhaltenen Pulver unter Zusatz von üblichen Hilfsstoffen Tabletten mit jeweils 200 mg Ibuprofengehalt hergestellt.

Unter in-vitro Bedingungen (900 ml künstlicher Magensaft/pH 1, 37°C) setzt ein Teilchenwachstum (BIFOQUELS on-line Particle-Sizer) unterhalb 13 Stunden ein.

**Beispiel 2**

Man stellt ein Nanosol wie in Beispiel 1 her, wählt jedoch eine Gelatine mit einem IEP von 3,6 aus, die bei einem pH von 2 ein Stabilitätsoptimum aufweist.

Unter den gleichen Prüfbedingungen (Beispiel 1) setzt ein Teilchenwachstum des erhaltenen Nanosols nach durchschnittlich 15 Stunden ein.

**Beispiel 3**

Arzneistoff: R-Flurbiprofen, enantiomerreine Wirkstoffsäure
Gelatinetyp: Typ B, 120 Bloom, Herstellung Beispiel II
Nanosol-Herstellung: analog Verfahren III
Gewichtsverhältnis Gelatine/Arzneistoff: 5 : 1
Der Arbeits-pH-Bereich für R-Flurbiprofen liegt unterhalb seines $pK_S$-Wertes von 4,16.
Der erfindungsgemäße Vortest und die Meßreihe ergibt bei einem pH-Wert von 3,2 für den isoionischen Ladungszusatnd ein Optimum mit einer Gelatine Typ B (IEP 4,5).

10 kg einer 6%igen wäßrigen Gelatinelösung werden mit destilliertem Wasser bei 40°C hergestellt und darin 15 g PVP K 15 aufgelöst. Durch Zugabe von Salzsäure (15%ig) wird auf pH 3,2 eingestellt.

120 g R-Flurbiprofen werden in 700 ml Isopropanol gelöst. Beide Lösungen werden homogen vermischt und das organische Lösungsmittel unter Vakuum entfernt.

Das entstandene Nanosol wird sprühgetrocknet und wie üblich zu Tabletten mit 50 mg R-Flurbiprofengehalt verpreßt.

Die Tabletten ergeben im Dissolutiontest nach USP XXII (900 ml Wasser, paddle, 65 rpm, 37°C) eine vollständige Auflösung unterhalb von 15 Minuten.

**Beispiel 4**

Eine Tablette, die unter gleichen Bedingungen wie in Beispiel 3 hergestellt wird, jedoch keinen Zusatz von PVP K 15 enthält, zeigt eine vollständige Auflösung unter gleichen Testbedingungen unterhalb von 25 Minuten.

**Beispiel 5**

Arzneistoff: R-Flurbiprofen, enantiomerreine Wirkstoffsäure
Gelatinetyp: handelsüblich, Typ B, 190 Bloom
Nanosol-Herstellung: analog Verfahren III
Gewichtsverhältnis Gelatine/Arzneistoff: 1,5 : 1
Die Herstellungsbedingungen des Nanosols entsprechen Beispiel 3, mit einer gleichgeladenen Gelatine, die jedoch eine höheren Bloomwert besitzt. Die Mengenverhältnisse sind wie folgt:
Gelatine: 150 g gelöst in 1,5 l destilliertem Wasser
R-Flurbiprofen: 100 g gelöst in 500 ml Isopropanol
PVP K 15: 15 g gelöst bei 40°C in der Gelatinelösung
Nach Entfernung des Isopropanols durch Evaporation wird das Nanosol lyophilisiert. Das in Hartgelatinekapseln mit jeweils 25 mg R-Flurbiprofen-Gehalt abgefüllte Nanosol löst sich in wäßrigem Medium unter den Prüfbedingungen wie in Beispiel 3 unterhalb von 5 Minuten vollständig auf.

Die so hergestellten Kapseln ergeben Blutspiegelmaximalwerte, die im Durchschnitt unterhalb einer Stunde liegen.

**Beispiel 6**

Arzneistoff: Ketoprofen (Racemat), Wirkstoffsäure
Gelatinetyp: Typ A, Kollagenhydrolysat, MG <$10^4$ D, Herstellung Beispiel I
Nanosol-Herstellung: analog Verfahren II
Gewichtsverhältnis Gelatine/Arzneistoff: 6 : 1
Der Arbeits-pH-Bereich für Ketoprofen liegt unterhalb des $pK_S$-Wertes von 5,3.
Der Schnelltest bei pH 4,5 zur Ermittlung der Oberflächenladung der Ketoprofenpartikel ergibt bei der Standardgelatine Typ B (IEP 3,5/200 Bloom) kein Nanosol. Unter gleichen Testbedingungen ergibt die Standardgelatine Typ A (IEP 9,5/200 Bloom) ein kurzzeitig stabiles Nanosol. Daher ist eine negative Oberflächenladung der Ketoprofenpartikel erkennbar.

In den anschließenden Versuchen zur Ermittlung des optimalen Kollagenhydrolysats hat sich ein Typ A, der durch Ionenaustauscher völlig von Fremdionen befreit ist und einen IEP von 8 besitzt, bei einem pH-Wert von 3,8 am besten geeignet.

Eine Lösung aus 300 g des oben beschriebenen Kollagenhydrolysats in 6 l Wasser wird bei 20°C hergestellt. 50 g Ketoprofen werden in 300 g Natronlauge 10% gelöst und zu der wässrigen Hydrolysatlösung gegeben. Nach intensiver Durchmischung wird die klare Lösung durch Zugabe einer abgemessenen Säuremenge auf einen pH-Wert von 3,8 gebracht. Das so hergestellte Nanosol weist zu 60% eine Partikelgröße kleiner als 410 nm auf.

Das Nanosol wird nach Aufkonzentrierung und Sprühtrocknung zu Tabletten mit jeweils 50 mg Ketoprofengehalt verarbeitet (Direkttablettierung).

Unter den Prüfbedingungen wie in Beispiel 3 löst sich die Tablette innerhalb von 10 Minuten vollständig auf.

**Beispiel 7**

Analog Beispiel 6 wird das so erhaltene Pulver granuliert und als Granulat mit einer Dosierung von 50 mg Ketoprofen einzeln abgefüllt. Das Granulat löst sich bei 20°C in wäßrigem Medium innerhalb von 3 Minuten vollständig auf.

Das erhaltene Granulat kann auch als Initialdosis in Kombination mit einer Nanosol-Matrix-Tablette oder herkömmlichen Retardformen in einer Hartgelatinekapsel vorliegen.

**Patentansprüche**

1. Akut-Arzneimittel zur Behandlung von schmerzhaften und/oder entzündlichen, sowie fieberhaften Erkrankungen, enthaltend ein pharmakologisch wirksames 2-Arylpropionsäurederivat in Form eines pharmazeutisch applizierbaren Nanosols, das als Träger Gelatine, ein Kollagenhydrolysat oder ein Gelatinederivat neben üblichen pharmazeutischen Hilfsstoffen enthält.

2. Arzneimittel nach Anspruch 1, dadurch gekennzeichnet, daß das Nanosol
   a) eine innere Phase aus dem 2-Arylpropionsäurederivat, das eine Teilchengröße von 10 - 800 nm aufweist und eine Oberflächenladung besitzt,
   b) eine äußere Phase aus Gelatine, einem Kollagenhydrolysat oder einem Gelatinederivat, welche(s) gegensinnig geladen ist,
   c) einen angenäherten oder vollständigen isoionischen Ladungszustand der inneren und äußeren Phase aufweist, und
   d) physiologisch resorbierbar ist.

3. Arzneimittel nach Anspruch 1 und/oder 2, dadurch gekennzeichnet, daß das 2-Arylpropionsäurederivat als Racemat oder als Gemisch aus dem Racemat und seinen Enantiomeren vorliegt.

4. Arzneimittel nach Anspruch 1 und/oder 2, dadurch gekennzeichnet, daß das 2-Arylpropionsäurederivat als Pseudoracemat vorliegt.

5. Arzneimittel nach Anspruch 1 und/oder 2, dadurch gekennzeichnet, daß das 2-Arylpropionsäurederivat als reines S-Enantiomer oder als reines R-Enantiomer vorliegt.

6. Arzneimittel nach Anspruch 1 und/oder 2, dadurch gekennzeichnet, daß das 2-Arylpropionsäurederivat als Mischung von S- und R-Enantiomeren vorliegt.

7. Arzneimittel nach einem der Ansprüche 1 bis 6, dadurch gekennzeichnet, daß das 2-Arylpropionsäurederivat als flüssige, wäßrige Nanodispersion vorliegt.

8. Arzneimittel nach einem der Ansprüche 1 bis 6, dadurch gekennzeichnet, daß das 2-Arylpropionsäurederivat als feste, resuspendierbare Nanodispersion vorliegt.

9. Arzneimittel nach einem der Ansprüche 1 bis 8, dadurch gekennzeichnet, daß das 2-Arylpropionsäurederivat eine durchschnittliche Teilchengröße unterhalb von 400 nm aufweist.

10. Arzneimittel nach einem der Ansprüche 1 bis 9, gekennzeichnet durch eine äußere Phase des Nanosols, die zusätzlich Polyvinylpyrrolidon in einem Gewichtsverhältnis von Gelatine zu Polyvinylpyrrolidon wie 5:1 bis 500:1, bevorzugt 10:1 bis 50:1, enthält.

11. Arzneimittel nach einem der Ansprüche 1 bis 10, dadurch gekennzeichnet, daß das 2-Arylpropionsäurederivat Ketoprofen ist.

12. Arzneimittel nach einem der Ansprüche 1 bis 10, dadurch gekennzeichnet, daß das 2-Arylpropionsäurederivat S-Naproxen ist.

13. Verfahren zur Herstellung eines kolloid-dispersen Systems eines 2-Arylpropionsäurederivates, dadurch gekennzeichnet, daß man
    a) eine Gelatine, ein Kollagenhydrolysat oder ein Gelatinederivat nach ihrem (seinem) isoelektrischen Punkt (IEP) so auswählt, daß ihr (sein) IEP mit dem Ladungszustand der 2-Arylpropionsäurederivatpartikel so abgestimmt ist, daß die Gelatine, das Kollagenhydrolysat oder ihr Derivat bei einem bestimmten pH-Wert mit dem ungelösten 2-Arylpropionsäurederivat zu Ladungsneutralität führt,
    b) die Gelatine, das Kollagenhydrolysat oder ihr Derivat in die wäßrige Solform überführt,

14

c) den pH-Wert in Abhängigkeit von dem IEP der Gelatine auf einen solchen Wert einstellt, daß die sich bildenden Nanopartikel des 2-Arylpropionsäurederivates annähernd oder vollständig ladungsneutral stabilisiert werden, und

d) vor oder nach der Stufe c) das 2-Arylpropionsäurederivat in dem wäßrigen Gelatinesol löst oder eine Lösung des 2-Arylpropionsäurederivates mit dem wäßrigen Gelatinesol vereinigt.

14. Verfahren nach Anspruch 13, dadurch gekennzeichnet, daß man in Stufe d) das gelöste 2-Arylpropionsäurederivat vor der Vereinigung mit dem wäßrigen Gelatinesol kurzzeitig in kolloid-disperse Form von Nanopartikeln überführt und die so erhaltene Dispersion von Nanopartikeln kontinuierlich mit dem wäßrigen Gelatinesol vereinigt.

15. Verfahren nach Anspruch 13 oder 14, dadurch gekennzeichnet, daß man
e1) das 2-Arylpropionsäurederivat in Form von Nanopartikeln ausfällt.

16. Verfahren nach Anspruch 13 oder 14, dadurch gekennzeichnet, daß man
e2) die in Stufe d) erhaltene kolloid-disperse Lösung sprühtrocknet oder gefriertrocknet und so ein stabiles resuspendierbares Nanosol erhält, daß nach Wiederauflösung in wäßrigem Medium ein kolloid-disperses System in Nanosolform ergibt.

17. Verfahren nach den Ansprüchen 13 bis 16, dadurch gekennzeichnet, daß man in Stufe d) das 2-Arylpropionsäurederivat in einem mit Wasser mischbaren organischen Lösungsmittel gelöst, zusetzt.

18. Verfahren nach einem der Ansprüche 13 bis 17, dadurch gekennzeichnet, daß man das 2-Arylpropionsäurederivat in sein Salz überführt.

19. Verfahren nach einem der Ansprüche 13 bis 17, dadurch gekennzeichnet, daß man die mit Wasser mischbaren organischen Lösungsmittel in Stufe b) dem wäßrigen Gelatinesol zusetzt und in Stufe d) das 2-Arylpropionsäurederivat in fester Form dieser Mischung zusetzt und damit löst.

20. Verfahren nach Anspruch 17 oder 19, dadurch gekennzeichnet, daß man das organische Lösungsmittel anschließend wieder entfernt.

21. Verfahren nach einem der Ansprüche 13 bis 15, dadurch gekennzeichnet, daß man die Nanopartikel-Lösung anschließend trocknet.

22. Verfahren nach Anspruch 21, dadurch gekennzeichnet, daß man die Lösung sprühtrocknet.

23. Verfahren nach Anspruch 21, dadurch gekennzeichnet, daß man die Lösung gefriertrocknet.

24. Verfahren nach Anspruch 14 oder 15, dadurch gekennzeichnet, daß man vor Einstellung des pH-Wertes auf den isoionischen Punkt in Stufe c) den pH-Wert so einstellt, daß das 2-Arylpropionsäurederivat ein Salz bildet.

25. Verfahren nach einem der Ansprüche 13 oder 14, dadurch gekennzeichnet, daß man im Anschluß an Stufe d) ein mit Wasser mischbares organisches Lösungsmittel zur Lockerung der Hydrathülle der Gelatinemoleküle zusetzt.

26. Verfahren nach Anspruch 25, dadurch gekennzeichnet, daß man einen Alkohol zusetzt.

27. Verfahren nach Anspruch 14, dadurch gekennzeichnet, daß man die kolloiden Teilchen kontinuierlich mit einer einstellbaren Partikelgröße herstellt.

28. Verfahren nach Anspruch 27, dadurch gekennzeichnet, daß man die Teilchengröße kontinuierlich mißt.

29. Verfahren nach einem der Ansprüche 13 bis 28, dadurch gekennzeichnet, daß man es kontinuierlich durchführt.

# EP 0 615 440 B1

**30.** Verfahren nach einem der Ansprüche 17 bis 29, dadurch gekennzeichnet, daß man in Stufe b) zusätzlich Polyvinylpyrrolidon im Verhältnis zu der Gelatine von 1:5 bis 1:500, bevorzugt 1:10 bis 1:50 zugibt.

**31.** Verfahren nach einem der Ansprüche 1 bis 30, dadurch gekennzeichnet, daß man als Gelatine eine Gelatine mit einem Maximum der Molekulargewichtsverteilung im Bereich von $10^4 - 10^7$ D einsetzt.

**32.** Verfahren nach einem der Ansprüche 1 bis 30, dadurch gekennzeichnet, daß man als Gelatine eine Gelatine mit einem Maximum der Molekulargewichtsverteilung unterhalb von $10^5$ D (mittel- bis niedrig-bloomig) einsetzt.

**33.** Verfahren nach Anspruch 32, dadurch gekennzeichnet, daß man eine Gelatine mit einer Molekularge-wichtsverteilung von $10^4 - 9{,}5 \times 10^4$ D einsetzt.

**34.** Verfahren nach einem der Ansprüche 1 bis 32, dadurch gekennzeichnet, daß die Lösegeschwindigkeit der äußeren Phase durch Gefrier- oder Sprühtrocknung erhöht wird.

**35.** Verfahren nach einem der Ansprüche 13 bis 30 und 33, dadurch gekennzeichnet, daß man für die Bildung von Komprimaten eine Gelatine mit 0 - 50 Bloom einsetzt.

**36.** Verfahren nach einem der Ansprüche 13-35, dadurch gekennzeichnet, daß man das 2-Arylpropionsäurederivat als Racemat einsetzt.

**37.** Verfahren nach einem der Ansprüche 13-35, dadurch gekennzeichnet, daß man das 2-Arylpropionsäurederivat als Racemat in Mischung mit seinen Enantiomeren einsetzt.

**38.** Verfahren nach einem der Ansprüche 13-35, dadurch gekennzeichnet, daß man das 2-Arylpropionsäurederivat als Pseudoracemat einsetzt.

**39.** Verfahren nach einem der Ansprüche 13-35, dadurch gekennzeichnet, daß man das 2-Arylpropionsäurederivat als reines S-Enantiomer oder reines R-Enantiomer einsetzt.

**40.** Verfahren nach einem der Ansprüche 13-35, dadurch gekennzeichnet, daß man das 2-Arylpropionsäurederivat als Mischung von S- und R-Enantiomeren einsetzt.

## Claims

**1.** Immediate-effect medicament for the treatment of painful and/or inflammatory, and also febrile disorders, containing a pharmacologically active 2-arylpropionic acid derivative in the form of a pharmaceutically administrable nanosol which, as excipient, contains gelatin, a collagen hydrolysate or a gelatin derivative in addition to customary pharmaceutical auxiliaries.

**2.** Medicament according to Claim 1, characterized in that the nanosol
a) has an inner phase of the 2-arylpropionic acid derivative which has a particle size of 10-800 nm and possesses a surface charge,
b) an outer phase of gelatin, a collagen hydrolysate or a gelatin derivative which is oppositely charged,
c) a nearly or completely isoionic state charge of the inner and outer phase, and
d) is physiologically absorbable.

**3.** Medicament according to Claims 1 and/or 2, characterized in that the 2-arylpropionic acid derivative is present as a racemate or as a mixture at the racemate and its enantiomers.

**4.** Medicament according to Claims 1 and/or 2, characterized in that the 2-arylpropionic acid derivative is present as a pseudoracemate.

**5.** Medicament according to Claims 1 and/or 2, characterized in that the 2-arylpropionic acid derivative is present as a pure S-enantiomer or as a pure R-enantiomer.

16

6. Medicament according to Claims 1 and/or 2, characterized in that the 2-arylpropionic acid derivative is present as a mixture of S- and R-enantiomers.

7. Medicament according to one of Claims 1 to 6, characterized in that the 2-arylpropionic acid derivative is present as a liquid, aqueous nanodispersion.

8. Medicament according to one of Claims 1 to 6, characterized in that the 2-arylpropionic acid derivative is present as a solid, resuspendable nanodispersion.

9. Medicament according to one of Claims 1 to 8, characterized in that the 2-arylpropionic acid derivative has an average particle size of below 400 nm.

10. Medicament according to one of Claims 1 to 9, characterized by an outer phase of the nanosol which additionally contains polyvinylpyrrolidone in a weight ratio of gelatin to polyvinylpyrrolidone such as 5:1 to 500:1, preferably 10:1 to 50:1.

11. Medicament according to one of Claims 1 to 10, characterized in that the 2-azylpropionic acid derivative is ketoprofen.

12. Medicament according to one of Claims 1 to 10, characterized in that the 2-arylpropionic acid derivative is S-naproxen.

13. Process for the preparation of a colloidally disperse system of a 2-arylpropionic acid derivative, characterized in that

a) a gelatin, a collagen hydrolysate or a gelatin derivative is selected according to its isoelectric point (IEP) such that its IEP in coordinated with the charge state of the 2-arylpropionic acid derivative particles, leading the gelatin, the collagen hydrolysate or its derivative to charge neutrality with the undissolved 2-arylpropionic acid derivative at a specific pH,

b) the gelatin, the collagen hydrolysate or its derivative is converted into the aqueous sol form,

c) the pH is adjusted as a function of the IEP of the gelatin to a value such that the nanoparticles of the 2-arylpropionic acid derivative formed are nearly or completely stabilized in neutrally charged form, and

d) before or after stage c) the 2-aryl-propionic acid derivative is dissolved in the aqueous gelatin sol or a solution of the 2-arylpropionic acid derivative is combined with the aqueous gelatin sol.

14. Process according to Claim 13, characterized in that in stage d) the dissolved 2-arylpropionic acid derivative is converted into a colloidally disperse form of nanoparticles briefly before combination with the aqueous gelatin sol and the dispersion of nanoparticles thus obtained is continuously combined with the aqueous gelatin sol.

15. Process according to Claim 13 or 14, characterized in that e1) the 2-arylpropionic acid derivative precipitates in the form of nanoparticles.

16. Process according to Claim 13 or 14, characterized in that e2) the colloidally disperse solution obtained in stage d) is spray-dried or freeze-dried and thus a stable resuspendable nanosol is obtained which after redissolution in aqueous medium yields a colloidally disperse system in nanosol form.

17. Process according to Claims 13 to 16, characterized in that in stage d) the 2-arylpropionic acid derivative is added dissolved in a water-miscible organic solvent.

18. Process according to one of Claims 13 to 17, characterized in that the 2-arylpropionic acid derivative is converted into its salt.

19. Process according to one of Claims 13 to 17, characterized in that the water-miscible organic solvent in stage b) is added to the aqueous gelatin sol and in stage d) the 2-arylpropionic acid derivative is added to this mixture in solid form and thus dissolved.

20. Process according to Claim 17 or 19, characterized in that the organic solvent is then removed again.

21. Process according to one of Claims 13 to 15, characterized in that the nanoparticle solution is then dried.

22. Process according to Claim 21, characterized in that the solution is spray-dried.

23. Process according to Claim 21, characterized in that the solution is freeze-dried.

24. Process according to Claim 14 or 15, characterized in that, before adjustment of the pH to the isoionic point in stage c), the pH is adjusted such that the 2-arylpropionic acid derivative forms a salt.

25. Process according to one of Claims 13 or 14, characterized in that following stage d) a water-miscible organic solvent is added to loosen the hydration shell of the gelatin molecules.

26. Process according to Claim 25, characterized in that an alcohol in added.

27. Process according to Claim 14, characterized in that the colloidal particles are produced continuously with an adjustable particle size.

28. Process according to Claim 27, characterized in that the particle size is continuously measured.

29. Process according to one of Claims 13 to 28, characterized in that it is carried out continuously.

30. Process according to one of Claims 17 to 29, characterized in that in stage b) polyvinylpyrrolidone is additionally added in a ratio to the gelatin of 1 : 5 to 1 : 500, preferably 1 : 10 to 1 : 50.

31. Process according to one of Claims 1 to 30, characterized in that the gelatin employed is a gelatin having a maximum in the molecular weight distribution in the range $10^4$ - $10^7$ D.

32. Process according to one of Claims 1 to 30, characterized in that the gelatin employed is a gelatin having a maximum in the molecular weight distribution of less than $10^5$ D (medium- to low-bloom).

33. Process according to Claim 32, characterized in that a gelatin having a molecular weight distribution of $10^4$ - 9.5 x $10^4$ D is employed.

34. Process according to one of claims 1 to 32, characterized in that the solution rate of the eternal phase is increased by freeze- or spray-drying.

35. Process according to one of Claims 13 to 30 and 33, characterized in that a gelatin of 0 - 50 bloom is employed for the formation of tablets.

36. Process according to one of Claims 13-35, characterized in that the 2-arylpropionic acid derivative is employed as a racemate.

37. Process according to one of Claims 13-35, characterized in that the 2-arylpropionic acid derivative is employed as a racemate mixed with its enantiomers.

38. Process according to one of Claims 13-35, characterized in that the 2-arylpropionic acid derivative is employed as a pseudoracemate.

39. Process according to one of Claims 13-35, characterized in that the 2-arylpropionic acid derivative is employed as a pure S-enantiomer or pure R-enantiomer.

40. Process according to one of Claims 13-35, characterized in that the 2-arylpropionic acid derivative is employed as a mixture of S- and R-enantiomers.

**Revendications**

1. Médicament à action rapide destiné au traitement de maladies accompagnées de douleur et/ou d'inflammation ainsi que de fièvre, contenant un dérivé pharmacologiquement actif d'acide 2-arylpropionique sous forme d'un nanosol pharmaceutiquement applicable, qui contient comme support de la gélatine, un hydrolysat de collagène ou un dérivé de gélatine, à côté de substances auxiliaires pharmaceutiques classiques.

2. Médicament suivant la revendication 1, caractérisé en ce que le nanosol présente
   a) une phase interne formée du dérivé d'acide 2-arylpropionique, qui présente des diamètres de particules de 10 à 800 nm et qui possède une charge de surface,
   b) une phase externe formée de gélatine, d'un hydrolysat de collagène ou d'un dérivé de gélatine, qui porte une charge opposée,
   c) un état de charge presque isoionique ou tout à fait isoionique des phases interne et externe, et
   d) peut être résorbé physiologiquement.

3. Médicament suivant la revendication 1 et/ou la revendication 2, caractérisé en ce que le dérivé d'acide 2-arylpropionique se présente comme racémate ou comme mélange du racémate et de ses énantiomères.

4. Médicament suivant la revendication 1 et/ou la revendication 2, caractérisé en ce que le dérivé d'acide 2-arylpropionique se présente comme pseudoracémate.

5. Médicament suivant la revendication 1 et/ou la revendication 2, caractérisé en ce que le dérivé d'acide 2-arylpropionique se présente comme S-énantiomère pur ou comme R-énantiomère pur.

6. Médicament suivant la revendication 1 et/ou la revendication 2, caractérisé en ce que le dérivé d'acide 2-arylpropionique se présente comme mélange de S- et R-énantiomères.

7. Médicament suivant l'une des revendications 1 à 6, caractérisé en ce que le dérivé d'acide 2-arylpropionique se présente comme nanodispersion aqueuse liquide.

8. Médicament suivant l'une des revendications 1 à 6, caractérisé en ce que le dérivé d'acide 2-arylpropionique se présente comme nanodispersion solide pouvant être remise en suspension.

9. Médicament suivant l'une des revendications 1 à 8, caractérisé en ce que le dérivé d'acide 2-arylpropionique présente un diamètre moyen de particules inférieur à 400 nm.

10. Médicament suivant l'une des revendications 1 à 9, caractérisé par une phase externe du nanosol qui contient en outre de la polyvinylpyrrolidone dans un rapport en poids de la gélatine à la polyvinylpyrrolidone de 5:1 à 500:1, de préférence de 10:1 à 50:1.

11. Médicament suivant l'une des revendications 1 à 10, caractérisé en ce que le dérivé d'acide 2-arylpropionique est le ketoprofen.

12. Médicament suivant l'une des revendications 1 à 10, caractérisé en ce que le dérivé d'acide 2-arylpropionique est le S-naproxen.

13. Procédé de production d'une préparation d'un dérivé d'acide 2-arylpropionique en dispersion colloïdale, caractérisé en ce que
    a) on choisit une gélatine, un hydrolysat de collagène ou un dérivé de gélatine d'après son point isoélectrique (PIE) de manière que son PIE concorde avec l'état de charge des particules de dérivé d'acide 2-arylpropionique à tel point que la gélatine, l'hydrolysat de collagène ou son dérivé conduise à la neutralité de charge avec le dérivé d'acide 2-arylpropionique non dissous à une valeur déterminée de pH,
    b) on fait prendre à la gélatine, à l'hydrolysat de collagène ou à son dérivé la forme d'un sol aqueux,

c) on règle la valeur du pH en fonction du PIE de la gélatine à une valeur telle que les nanoparticules du dérivé d'acide 2-arylpropionique qui se forment soient stabilisées en atteignant presque ou en atteignant totalement la neutralité de charge, et

d) avant ou après l'étape c), on dissout le dérivé d'acide 2-arylpropionique dans le sol aqueux de gélatine ou bien on réunit une solution du dérivé d'acide 2-arylpropionique et le sol aqueux de gélatine.

14. Procédé suivant la revendication 13, caractérisé en ce que dans l'étape d), le dérivé d'acide 2-arylpropionique dissous est brièvement transformé en une dispersion colloïdale de nanoparticules avant sa réunion avec le sol aqueux de gélatine et la dispersion de nanoparticules ainsi obtenue et le sol aqueux de gélatine sont réunis en continu.

15. Procédé suivant la revendication 13 ou 14, caractérisé en ce que e1) on précipite le dérivé d'acide 2-arylpropionique sous forme de nanoparticules.

16. Procédé suivant la revendication 13 ou 14, caractérisé en ce que e2) on sèche par pulvérisation ou par congélation la solution en dispersion colloïdale obtenue dans l'étape d) et on obtient de la sorte un nanosol stable pouvant être remis en suspension, qui donne après redissolution en milieu aqueux un système en dispersion colloïdale sous forme de nanosol.

17. Procédé suivant les revendications 13 à 16, caractérisé en ce qu'on ajoute le dérivé d'acide 2-arylpropionique dissous dans un solvant organique miscible à l'eau dans l'étape d).

18. Procédé suivant l'une des revendications 13 à 17, caractérisé en ce qu'on transforme le dérivé d'acide 2-arylpropionique en son sel.

19. Procédé suivant l'une des revendications 13 à 17, caractérisé en ce qu'on ajoute les solvants organiques miscibles à l'eau au sol de gélatine dans l'étape b) et on ajoute à ce mélange et on dissout donc le dérivé d'acide 2-arylpropionique sous forme solide dans l'étape d).

20. Procédé suivant la revendication 17 ou 19, caractérisé en ce qu'on chasse ensuite le solvant organique.

21. Procédé suivant l'une des revendications 13 à 15, caractérisé en ce qu'on sèche ensuite la solution de nanoparticules.

22. Procédé suivant la revendication 21, caractérisé en ce qu'on sèche la solution par pulvérisation.

23. Procédé suivant la revendication 21, caractérisé en ce qu'on sèche la solution par congélation.

24. Procédé suivant la revendication 14 ou 15, caractérisé en ce que, avant le réglage de la valeur du pH au point isoionique dans l'étape c), on ajuste la valeur du pH de manière que le dérivé d'acide 2-arylpropionique forme un sel.

25. Procédé suivant l'une des revendications 13 ou 14, caractérisé en ce que, à la suite de l'étape d), on ajoute un solvant organique miscible à l'eau pour affaiblir l'enveloppe d'hydrate des molécules de gélatine.

26. Procédé suivant la revendication 25, caractérisé en ce qu'on ajoute un alcool.

27. Procédé suivant la revendication 14, caractérisé en ce que les particules colloïdales sont produites en continu avec un diamètre de particules réglable.

28. Procédé suivant la revendication 27, caractérisé en ce qu'on mesure en continu le diamètre des particules.

29. Procédé suivant l'une des revendications 13 à 28, caractérisé en ce qu'il est mis en oeuvre en continu.

**30.** Procédé suivant l'une des revendications 17 à 29, caractérisé en ce qu'on ajoute en outre dans l'étape b) de la polyvinylpyrrolidone dans un rapport avec la gélatine de 1:5 à 1:500, de préférence de 1:10 à 1:50.

**31.** Procédé suivant l'une des revendications 1 à 30, caractérisé en ce qu'on utilise comme gélatine une gélatine ayant un maximum de distribution du poids moléculaire dans la plage de $10^4$ à $10^7$ D.

**32.** Procédé suivant l'une des revendications 1 à 30, caractérisé en ce qu'on utilise comme gélatine une gélatine ayant un maximum de distribution de poids moléculaire inférieur à $10^5$ D (degré Bloom moyen à faible).

**33.** Procédé suivant la revendication 32, caractérisé en ce qu'on utilise une gélatine ayant une distribution de poids moléculaire de $10^4$ à $9,5 \times 10^4$ D.

**34.** Procédé suivant l'une des revendications 1 à 32, caractérisé en ce que la vitesse de dissolution de la phase externe est accrue par lyophilisation ou séchage par pulvérisation.

**35.** Procédé suivant l'une des revendications 13 à 30 et 33, caractérisé en ce qu'on utilise pour la formation de comprimés une gélatine de 0 à 50° Bloom.

**36.** Procédé suivant l'une des revendications 13 à 35, caractérisé en ce qu'on utilise le dérivé d'acide 2-arylpropionique sous forme de racémate.

**37.** Procédé suivant l'une des revendications 13 à 35, caractérisé en ce qu'on utilise le dérivé d'acide 2-arylpropionique sous forme de racémate en mélange avec ses énantiomères.

**38.** Procédé suivant l'une des revendications 13 à 35, caractérisé en ce qu'on utilise le dérivé d'acide 2-arylpropionique sous forme de pseudoracémate.

**39.** Procédé suivant l'une des revendications 13 à 35, caractérisé en ce qu'on utilise le dérivé d'acide 2-arylpropionique sous forme du S-énantiomère pur ou du R-énantiomère pur.

**40.** Procédé suivant l'une des revendications 13 à 35, caractérisé en ce qu'on utilise le dérivé d'acide 2-arylpropionique sous forme de mélange des S- et R-énantiomères.

Fig. 1

Gelatinetyp A

Bereich der IEP´s

Ladungsverteilungen in den Gelatinetypen A (sauer) und B (alkalisch)

IEP = isoelektrischer Punkt

Fig. 2

GASTROINTESTINALTRAKT – wäßrig –

Magen pH ca. 1 – 3

Darm pH ca. 5 – 8

MUCUS

– hochviskos –

GLYKOCALIX

– negativ geladen –

GASTROINTESTINALMEMBRAN

– lipophil –

BLUTKREISLAUF

Arzneistoff – gelöst –

RESORPTION

Verteilung im Körper